# EUROPEAN PATENT APPLICATION

(11) **EP 3 260 170 A1**
(43) Date of publication of application: **27.12.2017**
(21) Application number: 16382285.1
(22) Date of filing: 20.06.2016
(51) Int. Cl.: A61Q 17/00, A61K 36/899, A61Q 19/00, A61K 8/97, A61P 17/16

(54) **USE OF EXTRACTS OF DESCHAMPSIA ANTARCTICA FOR COUNTERACTING HUMAN SKIN BARRIER DAMAGE CAUSED BY ENVIRONMENTAL AGGRESSIONS**

(71) Applicant: Industrial Farmaceutica Cantabria, S.A., 39011 Santander (ES)
(72) Inventor: MATJI TUDURI, José Antonio, E-28043 Madrid (ES); BRIEVA DELGADO, Aurora, E-28027 Madrid (ES); DOMÍNGUEZ VALDÉS-HEVIA, Marta, E-28043 Madrid (ES); GUERRERO GÓMEZ-PAMO, Antonio, E-28007 Madrid (ES); RAMÍREZ DIÉGUEZ, Alain, E-28810 Villalbilla, Madrid (ES); BERETTI, Costanzo, 25135 Brescia (IT); GONZÁLEZ RODRÍGUEZ, Salvador, E-28891 Velilla de San Antonio, Madrid (ES); PIVEL RANIERI, Juan Pablo, E-28224 Pozuelo de Alarcón, Madrid (ES)
(74) Representative: ABG Patentes, S.L.

(57) **Abstract**

The invention refers to the use of extracts of *Deschampsia antarctica* (EDA) for counteracting the human skin barrier damage caused by environmental aggressions and for preventing and/or ameliorating disorders related to an impaired barrier function.

## Description

### FIELD OF THE INVENTION

The present invention relates to the field of cosmetics or dermatology. More particularly, the invention refers to the use, either therapeutic or cosmetic, of extracts of *Deschampsia antarctica* (EDA) for counteracting the human skin barrier damage caused by environmental aggressions and for preventing and/or ameliorating related disorders.

### BACKGROUND

Air pollution is an increasing concern worldwide. The rise of air pollution levels over the years, especially in the urban areas, has major effects on the skin. Climatic imbalances are also known to have a negative effect on the skin.

Skin is composed of three superposed layers that are, from surface to body depth, epidermis, dermis and hypodermis.

Epidermis is an epithelium, that can be conventionally divided, according to morphological and histological criteria, into four layers, from the deepest to the outermost: the basal lamina and the spinosal layer (who form the deep epidermis), and the granular layer (superficial epidermis) and the corneal layer (or stratum corneum). Epidermis is in contact with external environment.

The skin, and mainly the upper layer of the epidermis, constitutes a barrier against external attacks, in particular chemical, mechanical or infectious attacks, and, therefore, a number of defensive reactions against environmental factors (climate, ultraviolet rays, pollutants, and the like) and/or xenobiotic, such as, for example, microorganisms, occur therein. This property, referred to as barrier function, is mainly provided by the most superficial layer of the epidermis, namely the stratum corneum (also known as horny layer).

Thus, a detrimental change in the cutaneous barrier can occur in the presence of external attacks, such as irritants (detergents, acids, bases, oxidizing agents, reducing agents, concentrated solvents, toxic gases or smoke), mechanical stresses (rubbing, impacts, abrasion, tearing of the surface, emission of dust or other particles, shaving or depilation), climatic imbalances (cold, heat, wind, dryness, radiation) or xenobiotics (undesirable microorganisms, allergens), or of internal attacks, such as psychological stress. For instance, it has been described the impact of urban pollution on skin barrier function (Pan TL, Wang PW, Aljuffali IA, Huang CT, Lee CW, Fang JY. The impact of urban particulate pollution on skin barrier function and the subsequent drug absorption. J Dermatol Sci. 2015 Apr;78(1):51-60) or, in general, in skin health (Mancebo SE, Wang SQ. Recognizing the impact of ambient air pollution on skin health. J Eur Acad Dermatol Venereol. 2015 Dec;29(12):2326-32). In the particular case of atopic dermatitis (AD), Ahn review how a variety of air pollutants, such as environmental tobacco smoke, particulate matter, etc., act as risk factors for the development or aggravation of AD due to its ability to induce oxidative stress in the skin, leading to skin barrier dysfunction or immune dysregulation (Ahn K. The role of air pollutants in atopic dermatitis. J Allergy Clin Immunol. 2014 Nov;134(5):993-9; discussion 1000).

This detrimental change in the cutaneous barrier can be reflected in particular by cutaneous discomfort, sensory phenomena and in particular unpleasant phenomena, or also cutaneous dryness, which can in particular be measured by the imperceptible water loss. A person may then experience a feeling of cutaneous discomfort with, for instance, some of the following symptoms: smarting, tightness, a feeling of tautness, inflammation and/or itching. These feelings of cutaneous discomfort are more frequent in the most exposed areas of the body, namely the hands, feet, face and scalp.

One of the functions of the stratum corneum is to take up and retain the water contained in the epidermis, and any impairment of its structure and/or its function may result in changes in the hydration of the skin leading to an increase in transepidermal water loss. An excessive dry skin can lead in turn to an impairment of the skin barrier function and activation of skin barrier disorders such as atopic dermatitis, resulting in a vicious circle of reduced barrier quality and increased damage from environmental stress.

Although it is known that skin barrier deficiencies are complex conditions in which different causes may be involved, moisturizers or emollients are among the most common treatments since they are somewhat able to break the dry skin cycle and maintain the smoothness of the skin,

Cosmetics and pharmaceutical market is however still demanding new solutions for enhancing the skin barrier function and helping to protect against environmental aggressions and dryness.

*Deschampsia antarctica* Desv. (Poaceae) is the only native Gramineae found in the Antarctic. Mainly, it is located in Antarctic Peninsula and its offshore islands. This plant survives in a very harsh climate, therefore it has attracted scientists to search for the freeze-tolerance genes from this plant. Also *Deschampsia antarctica* has the special ability to not present photoinhibition in circumstances of overexposure to sunlight, due to protection of their enzymatic systems, exercised by various agents (such as antioxidants, "refolding" regulators, dehydrins, etc.) (Pérez-Torres E, Garcia A, Dinamarca J, Alberdi M, Gutiérrez A, Gidekel M, Ivanov AG, Hüner NPA, Corcuera LJ, Bravo L. The role of photochemical quenching and antioxidants in photoprotection of Deschampsia antarctica. Funct Plant Biol 2004, 31: 731-741).

WO2009064480 A1 discloses the antineoplastic activity from the Extracts of *Deschampsia antarctica* (EDA), which may be formulated in compositions of tablets and pellets for treating patients with cancer.

WO2010086464A1 reports the use of EDA as a new agent for skin photoprotection against UVA and UVB radiation. WO2011009977A2 proposes its use for the prevention of skin lesions, particularly non-melanoma skin cancer, caused by exposure to ultraviolet radiation. More recently, WO2013084193A2 relates to the therapeutic effects of the extract in the prevention of photoaging and cancer induced by ultraviolet radiation.

It has been postulated in the above prior art documents that the beneficial effects for the skin attributed to the EDA are mainly due to its antioxidant, anti-inflammatory and photoprotective activity. However, to the knowledge of the inventors, no prior art document discloses the capacity of EDA as skin protective agent against environmental aggressions not related to UV radiation that may cause or aggravate disorders related to an impaired barrier function.

There is a need for new solutions for counteracting the damages to the skin caused by environmental aggressions as well as preventing and/or ameliorating related skin barrier disorders.

The object of the present invention is to meet these needs.

### BRIEF DESCRIPTION OF THE INVENTION

The present invention solves the aforementioned needs since it relates to the new use of Extracts of *Deschampsia antarctica* (EDA) for counteracting the damages to the human skin barrier that can occur due to the effect of environmental aggressions. In particular, the inventors of the present invention have found *inter alia* that EDA exhibits a prominent protective effect on human skin cells against cutaneous dryness, osmotic and thermal stress, and several common air pollutants.

In the context of this invention, EDA can be used for application to healthy skin subjected to or which may be subjected to the influence of environmental aggressions such as climatic imbalances or pollutants and for this reason liable to display cutaneous discomfort or cutaneous dryness. In other specific cases, EDA can be applied to the skin when it already exhibits clinical signs of a pathological deficiency in the cutaneous barrier.

Therefore, one aspect of the present invention relates to the non-therapeutic or cosmetic use of EDA for counteracting the human skin barrier damage caused by environmental aggressions.

According to another aspect, the present invention relates to EDA for use in the amelioration and/or prevention of human skin barrier disorders and associated signs.

According to another aspect, the present invention relates to the use of EDA for the preparation of a composition intended for counteracting the human skin barrier damage caused by environmental aggressions and/or for preventing and/or ameliorating skin barrier disorders and associated signs.

According to another aspect, the present invention relates to a method for counteracting the human skin barrier damage caused by environmental aggressions, the method comprising administering to the subject in need thereof an effective amount of EDA.

According to another aspect, the present invention relates to a method for the ameliorationand/or prevention of human skin barrier disorders and associated signs, the method comprising administering to the subject in need thereof an effective amount of EDA.

A further aspect of the invention relates to a composition intended for counteracting the human skin barrier damage caused by environmental aggressions and/or for use in the treatment and/or amelioration of skin barrier disorders and associated signs, said composition comprising an effective amount of EDA in a physiologically acceptable medium.

These aspects and preferred embodiments thereof are additionally also defined in the claims.

### BRIEF DESCRIPTION OF THE FIGURES

**Figure 1** shows results obtained in example 1 with respect to the effect of pretreatment with EDA on cell death in dermal fibroblasts caused by dehydration.
**Figure 2** shows results obtained in example 1 with respect to the effect of pretreatment with EDA on cell death in dermal fibroblasts caused by hyperosmotic shock (sucrose 0.3 M).
**Figure 3** shows results obtained in example 3 with respect to the effect of pretreatment with EDA on cell viability of dermal fibroblasts that are subsequently exposed to tobacco smoke.
**Figure 4** shows a 6-well plate used in example 3 with respect to the effect of pretreatment with EDA on cell viability of dermal fibroblasts that are subsequently exposed to tobacco smoke.
**Figure 5** shows results obtained in example 3 with respect to the effect of pretreatment with EDA on cell morphology of dermal fibroblasts that are subsequently exposed to tobacco smoke (A: cells exposed to tobacco smoke, B: cells pretreated with EDA and exposed to tobacco smoke).
**Figure 6** shows results obtained in example 3 with respect to the effect of pretreatment with EDA on cell viability of keratinocytes (cell line HaCaT) that are subsequently exposed to tobacco smoke.
**Figure 7** shows the results obtained in example 4 with respect to the effect of exposure to Cd(II) on cell death in dermal fibroblasts.
**Figure 8** shows the results obtained in example 4 with respect to the effect of pretreatment with EDA on cell death in dermal fibroblasts caused by exposure to Cd(III).
**Figure 9** shows the results obtained in example 4 with respect to the effect of exposure to Cr(III) and Cr(VI) on cell death in dermal fibroblasts.

### DETAILED DESCRIPTION OF THE INVENTION

Skin interfaces with the environment and is the first line of defense from external, often hostile, environment, and the semipermeable epidermal barrier prevents both the escape of moisture and the entry of infectious or toxic substances.

Many individuals have a genetic predisposition to have deficiencies in the cutaneous barrier. Likewise, external factors such as climatic conditions and exposure to air pollution may lead to an impaired skin barrier function and thus causing or aggravating skin barrier disorders. Indeed, the real cause of those disorders that are related to damages in the skin barrier is not exactly known but it is likely caused by both genetic (runs in the family) and environmental factors. In particular, environmental aggressions (pollution, passive smoking, etc.) may explain the significant increase of cases of skin barrier deficiencies (dry skin, atopic skin, etc.) over the past few years and that people who live in cities appear more likely to get this disease.

Both physicians and consumers are increasingly concerned about the damaging effects climate imbalances (e.g. heat, cold, wind) and chemical air pollutants (e.g. smoke, heavy metals, polycyclic aromatic hydrocarbons (PAHs), volatile organic compounds (VOCs), oxides, particulate matter (PM) and ozone (Oₛ)) can have on the skin, and therefore effective skin-protection solutions are needed to protect skin against these aggressors.

Impairment of skin barrier function is often accompanied by an altered integrity of the stratum corneum and a consequential increase in transepidermal water loss. Thus, impaired barrier function and skin dryness are intimately inter-related.

A skin barrier disorder may be generally defined as a state in which the skin, or more particularly the epidermis and especially the horny layer, does not exhibit a normal barrier function. This deterioration of the skin may merely be a minor aesthetic alteration and/or a sensation of cutaneous discomfort or cutaneous dryness in a skin considered as healthy but also a pathological condition. Specific examples of skin barrier disorders are dry skin, atopic dermatitis, contact dermatitis and the like.

Dry skin, also called xerosis, is a very common skin abnormality. A dry skin has a rough feel, appears covered with squamae and manifests itself essentially through a sensation of tautness and/or tension.

Dry skin is in fact accompanied by a desquamation disorder and presents various stages as a function of the severity of this desquamation. When the skin is slightly dry, these squamae are abundant but sparingly visible to the naked eye, and removal takes place corneocyte by corneocyte. They are increasingly infrequent but increasingly visible to the naked eye when this disorder worsens, and the patches may comprise several hundred corneocytes, thus representing more or less large patches, known as squamae.

A skin suffering from skin dryness generally displays the following signs, namely a rough and flaky feel, and also decreased suppleness and elasticity. There are also studies that have demonstrated an incidence of dryness on the origin and formation of wrinkles and fine lines, and indeed, in visual terms a dry skin makes said wrinkles and fine lines more apparent.

Moreover, from a sensory viewpoint, skin dryness is characterized by a sensation of tautness and/or itching. For obvious reasons, these manifestations are not only sources of discomfort, or even pain, but also an unaesthetic appearance.

The origin of this dryness may be of constitutional or acquired type. In the case of acquired dry skin, the involvement of external parameters such as exposure to chemical agents, to harsh climatic conditions, to sunlight or else to certain therapeutic treatments (for example retinoids) is a determining factor. Under these external influences, the epidermis may then become momentarily and locally dry. This may concern any type of epidermis.

Indeed, skin is known to have tendency to drying due to environmental, psychological and hormonal factors. In particular, pollution is an increasing concern worldwide due to the industrialization and the release of high quantity of chemical compounds in the air. These toxic components adhere to the skin's surface and disturb the horny layer's integrity. This leads to many damages such as the loss of hydration. Climatic conditions such as cold or wind are also known to promote skin dryness.

In view of the increasing dangerous environmental factors, it is important nowadays that skin be well moisturized and does not suffer a water loss that would lead to skin wilting and drying. Therefore, treating and preventing cutaneous dryness has become essential, not only from a therapeutic viewpoint, but also from an aesthetic viewpoint.

Atopic dermatitis (AD), also referred to as atopic eczema, is a chronic disease of the skin in which the skin is dry, easily irritated, allergen predisposed, typically scaly, often thickened, commonly red, sometimes exudative, frequently infected and above all itchy. In industrialized countries, the prevalence rates in childhood range from 15 to 30 percent and in adults from 2 to 10 percent of the general population. Atopy appears to show a strong hereditary component, but as for other skin barrier disorders, environmental aggressions are also thought to play an important role in the development of atopy,

The inventors of the present invention have found that Extracts of *Deschampsia antarctica* (EDA) may be favorably used for enhancing the human skin barrier function and more particularly as skin protective agent against environmental aggressions not derived from UV radiation and cutaneous dryness.

Advantageously, EDA provides protection towards environmental aggressions not associated with UV radiation, either from the sun or from artificial sources (including UVA and UVB radiation), that may affect the human skin barrier function and may cause cutaneous dryness. These factors include exposure to air pollution, especially to chemical air pollutants [for instance smoke (such as tobacco smoke), heavy metals (such as arsenic, cadmium, chromium), polycyclic aromatic hydrocarbons (PAHs), volatile organic compounds (VOCs), oxides, particulate matter (PM), and ozone (O₃)] and climatic conditions other than UV radiation such as osmotic shocks, low air humidity, environmental thermal stress, etc.

The EDA under this invention may be extracted from the plant obtained from its native environment or from plants cultured in a controlled environment. Mainly, the plant grows naturally in the Antarctic Continent, which is a territory subject to strict regulations for its protection, which make its exploitation, and therefore the harvesting there of *Deschampsia antarctica* for commercial purposes impossible. It therefore becomes necessary to obtain it by cultivation outside of its natural habitat.

Different methods for obtaining extracts of *Deschampsia antarctica* are known in the state of the art. For instance, the extract of *Deschampsia antarctica* may be obtained by a procedure previously established by the authors, which makes use of an aqueous method. This avoids the use of organic solvents, which present contamination problems and residues that are difficult to eliminate from the extract. Thus, according to preferred embodiment, the extract is an aqueous extract of *Deschampsia antarctica.* So EDA represents the matter extracted from *Deschampsia antarctica.*

In a particular embodiment, dry green leaves (HVS, by its Spanish acronym) obtained from the plant are introduced in a percolator in which water or an aqueous solvent is circulating under controlled conditions of temperature and extraction time. These extracts that are essentially aqueous are then stabilized and dried under vacuum. The resulting powder is milled and sieved.

In a more particular embodiment, HVS is introduced in a percolator in which water or an aqueous solvent is circulating at a temperature of about 30 to 100 °C, with a ratio solvent / HVS of about 11/1 to 60/1, for a period of about 2 to 7 h. The liquid is collected and preferably filtered with a filter of about 10 to 0.45 µm, and then it is concentrated from 0 to 6 times. Next the resulting liquid may be lyophilized or dried by addition of excipients such as maltodextrin or starch and dried under vacuum (about 50 to 300 mbar) at a temperature of about 30 to 90 °C. The resulting powder is milled and sieved.

In the context of the present invention, the following terms have the meaning detailed below.

For the purpose of the present invention, the expression "effective amount" is intended to denote the minimum amount required for observation of the expected effect, i.e. a cosmetic effect or a therapeutic effect, it being understood that the effective amounts required for obtaining a cosmetic effect or a therapeutic effect may be, as appropriate, identical or different.

The term "subject", as used herein, refers to any human that is suffering from skin barrier damage or skin barrier disorder. Thus, the skin to be treated with EDA or a composition thereof is human skin.

For the purpose of the invention, the term "cosmetic" is intended to denote a use, composition or the like intended mainly for providing an aesthetic effect and/or comfort.

For the purpose of the invention, the term "therapeutic" is intended to denote a use, composition or the like intended for providing a prophylactic or curative effect with regard to skin and in particular epidermal disorders, recognized as reflecting a pathological state.

The uses, methods and compositions provided by the present invention may be therapeutic and/or cosmetic. Moreover, depending on the regulatory framework, EDA in accordance with the invention may be intended for the preparation of a composition under the category of "cosmeceuticals", i.e. a combination of cosmetics and pharmaceuticals, or more particularly, a cosmetic product with at least one biologically active ingredient which is recognized to have medical or drug-like benefits.

EDA or a composition thereof in accordance with the invention may in general be intended for improving the general state of the human skin and/or the scalp, and in particular, for counteracting the human skin barrier damage caused by environmental aggressions and for preventing and/or ameliorating human skin barrier disorders and associated signs.

In a particular embodiment, EDA or a composition thereof is intended for preventing and/or reducing discomfort, unpleasant phenomena or cutaneous dryness of human skin subjected to the influence of environmental aggressions not derived from UV radiation.

In another particular embodiment, EDA or a composition thereof is intended for preventing and/or ameliorating a human skin barrier disorder and associated signs, the skin barrier disorder being selected from dry skin, atopic dermatitis, contact dermatitis and the like.

The signs associated with human skin barrier disorders are intended to mean not only all the modifications of the outer appearance of the skin due in particular to the action of environmental aggressions other than UV radiation and the dehydration of the skin (in particular, epidermis), such as the rough and flaky appearance, and the decreased suppleness, but also the sensations or cutaneous discomfort caused, such as itching and/or tautness. It may, in fact, occur that these sensations are felt by an individual without any visual symptom necessarily being perceptible.

EDA or a composition thereof in accordance with the invention may in particular be intended for preventing and/or ameliorating skin dryness and/or skin disorders associated with a state of skin dryness, particularly dryness of an epidermis, and more particularly a defect of hydration of the stratum corneum.

EDA or a composition thereof in accordance with the invention may in particular be intended for improving tonicity and/or firmness and/or elasticity and/or softness of skin

EDA or a composition thereof in accordance with the invention may in particular be intended for restoring and/or enhancing osmotic balance in the cells of the skin and/or the scalp and/or for protecting the cells of the skin and/or the scalp from osmotic shocks.

EDA or a composition thereof in accordance with the invention may in particular be intended for treating sensitive skins.

The composition of the invention can be locally applied to the face, the neck, the neckline, the hands, the feet, the scalp or any other topographic area. Thus the present invention advantageously provides the possibility to proceed whenever necessary or desirable, to very localize and selective "soft" treatments thanks to the topical application method.

According to the invention, "physiologically acceptable medium" means, without limitation, an aqueous or hydroalcoholic solution, a water-in-oil emulsion, an oil-in-water emulsion, a microemulsion, an aqueous gel, an anhydrous gel, a serum or a vesicle dispersion. "Physiologically acceptable" means that the described compositions or compounds are suitable for use in contact with human scalp, hair, hairs and skin, without undue toxicity, incompatibility, instability, allergic response, and the like.

The compositions according to the invention may be in any galenic form such creams, lotions, milk or cream ointments, gels, emulsions, dispersions, solutions, suspensions, cleansers, foundations, anhydrous preparations (sticks, in particular lipbalm, body and bath oils), shower and bath gels, shampoos and scalp treatment lotions, cream or lotion for care of skin or hair, make-up removing lotions or creams, sun-screen lotions, milks or creams, artificial suntan lotions, creams or milks, foams, gels or lotions, make-up, skin "essences," serums, adhesive or absorbent materials, transdermal patches, or powders, emollient lotion, milk or cream, sprays, oils for the body and the bath, foundation tint bases, pomade, emulsion, colloid, compact or solid suspension, sprayable or brossable formulation, , facial or body powder, mousse, skin conditioners, moisturizers, hair sprays, soaps, body exfoliants, astringents, and so on. Compositions in accordance with the invention include cosmetics, personal care products, cosmoceuticals and pharmaceutical preparations (medicaments). One can also consider a composition in the shape of foam or in the form of compositions for aerosol also including a propellant agent under pressure.

It is possible to integrate in the composition of the invention products already known as having an activity helping, improving or restoring the barrier function of the skin and in particular enhancing cutaneous hydration. Thus, according to a particular embodiment, the composition of the present invention may further comprise at least one additional active ingredient selected from the group consisting of moisturizing agents, humectant agents, occlusive agents, desquaming agents, agents acting on the cutaneous barrier function, UV filters, agents regulating cellular homeostasy, film formers, agents regulating aquaporin expression, anti- aging agents, anti-wrinkle agents, anti- oxidizing agents, anti-inflammatory agents, depigmenting or propigmenting agents, self-taning agents, anti-glycation agents, NO-synthase inhibitors, agents stimulating dermal or epidermal macromolecule synthesis and/or preventing their degradation, agents stimulating fibroblast and/or keratinocytes proliferation or stimulating keratinocytes differentiation, anti-pollution and/or anti-radicals agents, agents acting on microcirculation, anti- stretch marks agents, agents acting on energetic metabolism of cells, anti-acne agents, and mixture thereof.

Non limiting examples of these agents are : betaine, glycerol, Actimoist Bio 2™ (Active Organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals Inc.), Hydra'Flow™ (Sochibo), Hydromoist L™ (Symrise), RenovHyal™ (Soliance), Seamoss™ (Biotech Marine), Essenskin™ (Sederma) and Moist 24™ (Sederma).

The following examples demonstrate as pretreatment with EDA protects fibroblasts and keratinocytes (two types of cells essential to the creation and maintenance of barrier function (Varkey M, Ding J, Tredget EE. Superficial dermal fibroblasts enhance basement membrane and epidermal barrier formation in tissue-engineered skin: implications for treatment of skin basement membrane disorders. Tissue Eng Part A. 2014 Feb;20(3-4):540-52. Lee SH, Jeong SK, Ahn SK. An Update of the Defensive Barrier Function of Skin. Yonsei Medical Journal 2006. 47: 293 - 306) from different environmental aggressions. These examples are merely illustrative of certain embodiments of the invention and cannot be considered as restricting it in any way.

### Examples

### Example 1 - Protective effect of pretreatment with EDA against dehydration and hyperosmotic shock

The object of this experiment is to evaluate the possible protective effect of pretreatment with *Deschampsia antarctica* extract in human fibroblasts against dehydration occurring either due to the absence of air moisture or hyperosmotic shock, measuring variations in cell survival.

### Materials and methods:

### - Products to be tested:

*Deschampsia antarctica* extract (EDA): In experiments with fibroblasts, EDA dried with maltodextrin, batch 160215, was used. The product was dissolved in a Dulbecco modified Eagle medium (DMEM) medium at a concentration of 10 mg/ml (equivalent to 2.1 mg of EDA/ml), stirred in a magnetic stirrer for 30 minutes and filtered through a 0.2 µm sterile cellulose acetate filter (Minisart, Sartorius).

### - Culture media and reagents:

Phenol red-free DMEM medium (Dulbecco's modified Eagle medium) (Sigma). DMEM medium (Sigma). Penicillin-Streptomycin (Sigma). Glutamine (Sigma). Fetal calf serum (Sigma). 0.25% Trypsin-Ethylen diamine tretraacetate (EDTA) (Sigma). Phosphate-buffered saline (PBS). Hank's balanced salt solution (hereinafter Hank's) (Sigma). Sucrose (Sigma). Glutaraldehyde (Sigma). Crystal violet (Sigma). Sodium dodecyl sulfate (SDS) (Sigma).

### - Fibroblast cell culture:

The cells used were human dermal fibroblasts isolated from the outer area of the retroauricular flap obtained from different healthy voluntary donors and preserved in liquid N₂. The experiments used cells grown in culture flasks containing DMEM supplemented with 2 mM glutamine, 50 µg/ml penicillin, 50 U/ml streptomycin, 10% fetal calf serum, in a Heraeus oven with an atmosphere of 7% CO₂ and 93% humidified air at 37°C, until reaching about 90% of confluence, after which a new pass was performed by means of trypsinization. For the experiments, cells between passes 9 and 11 which were seeded in 96-well plates with flat bottom (Nunc) at an initial density of 10⁵ cells/ml, respectively, were used and were left to grow in an incubator until reaching confluence.

### - Treatment with products:

Pretreatment with EDA: 100 µl per well of the EDA solution prepared as indicated above (or the relevant dilutions in complete DMEM) were added to half of each plate and the plate was incubated for about 24 hours in an incubator.

Dehydration: After pretreatment, the plates were washed twice with Hank's and the medium was removed or 100 µl per well of phenol red-free DMEM solution (controls) were added and the plates were incubated moisture-free for 90 minutes in an incubator at 37°C.

Hyperosmotic shock: After pretreatment, the plates were washed twice with Hank's and 100 µl per well of 0.3 M sucrose solution in DMEM (osmolarity: 600 mosM) or 100 µl per well of phenol red-free DMEM solution (controls. Osmolarity: 300 mosM) were added and they were incubated for 6 hours in an incubator at 37°C, 7% CO₂, 93% humidified air.

### - Measuring cell viability:

The plates were washed twice with Hank's. They were fixed with 100 µl of a 0.25% glutaraldehyde solution in Hank's for 15 minutes at room temperature. They were washed once with water. They were stained with a 0.2% crystal violet solution in a 2% ethanol solution for 15 minutes at 37°C. The plate was washed with water and the cells were solubilized with 100 µl of 1% sodium dodecyl sulfate (SDS). The plate was carefully stirred in a plate stirrer and the absorbance at 550 nm was read in a BMG Fluostar Optima spectrophotometer.

### - Statistical analysis:

Statistical analysis was performed for each experiment, considering the value of each well as an independent value, determining the significance by means of Student's t-test using Excel or Graph Pad Prism.

### Results:

### - Effect of pretreatment with EDA on cell death caused by dehydration:

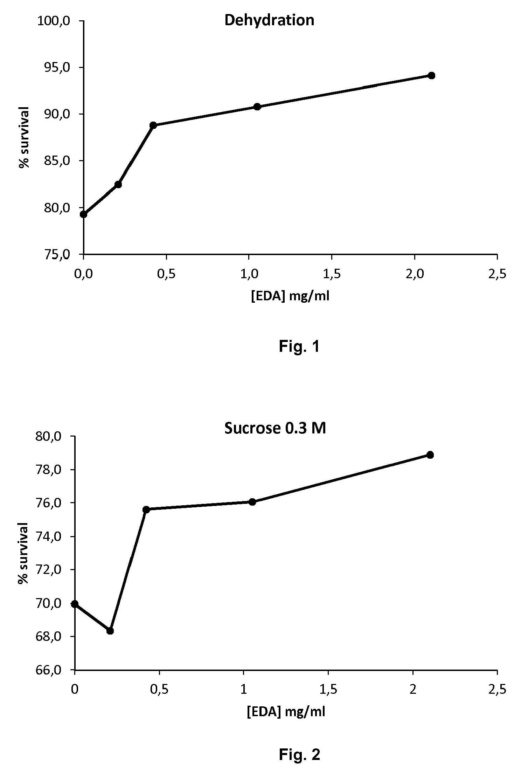

The following table shows the result of two different experiments conducted with fibroblasts from different donors and figure 1 shows the average of survival of both experiments.

| | **n** | **x̅** | **SD** | **CV** | **% surv.** | **P** |
|---|---|---|---|---|---|---|
| Non-dehydrated control cells | 59 | 0.363 | 0.021 | 5.7 | 100.0 | |
| Cells dehydrated 90 min | 59 | 0.296 | 0.014 | 4.8 | 81.6 | **2.4E-40** vs Non-dehydrated control cells (CC) |
| Dehydrated cells pretreated with EDA 0.21 mg/ml | 30 | 0.301 | 0.028 | 9.4 | 82.9 | 0.4106 vs dehydrated cells. |
| Dehydrated cells pretreated with EDA 0.42 mg/ml | 29 | 0.317 | 0.030 | 9.3 | 87.4 | **8.7E-04** vs dehydrated cells. |
| Dehydrated cells pretreated with EDA 1.05 mg/ml | 30 | 0.341 | 0.019 | 5.6 | 94.0 | **4.3E-15** vs dehydrated cells. |
| Dehydrated cells pretreated with EDA 2.1 mg/ml | 29 | 0.349 | 0.022 | 6.3 | 96.3 | **1.0E-14** vs dehydrated cells. |

| | **n** | **x̅** | **SD** | **CV** | **% surv.** | **P** |
|---|---|---|---|---|---|---|
| Non-dehydrated cells | 54 | 0.37 | 0.02 | 6.3 | 100.0 | |
| Cells dehydrated 90 min | 59 | 0.28 | 0.02 | 5.8 | 77.0 | **5.7E-43** vs control cells (CC) |
| Dehydrated cells pretreated with EDA 0.21 mg/ml | 30 | 0.3 | 0.020 | 6.7 | 82.1 | **1.5E-05** vs dehydrated cells |
| Dehydrated cells pretreated with EDA 0.42 mg/ml | 29 | 0.33 | 0.02 | 6.4 | 90.2 | **1.7E-19** vs dehydrated cells |
| Dehydrated cells pretreated with EDA 1.05 mg/ml | 30 | 0.32 | 0.02 | 7.0 | 87.6 | **1.6E-14** vs dehydrated cells |
| Dehydrated cells pretreated with EDA 2.1 mg/ml | 29 | 0.34 | 0.02 | 5.1 | 92.0 | **1.3E-24** vs dehydrated cells |

As can be seen, pretreatment with EDA has a significant protective effect against cell death induced by dehydration.

### - Effect of pretreatment with EDA on cell death caused by hyperosmotic shock:

The following table shows the result of two different experiments conducted with fibroblasts from different donor and figure 2 shows the average of survival of both experiments.

| | **n** | **x̅** | **SD** | **CV** | **% surv.** | **P** |
|---|---|---|---|---|---|---|
| Sucrose-free control cells | 59 | 0.482 | 0.042 | 8.7 | 100.0 | |
| Cells with 0.3 M sucrose | 59 | 0.334 | 0.020 | 6.0 | 69.4 | **2.5E-47** vs sucrose-free control cells (CC) |
| Cells with 0.3 M sucrose pretreated with 0.21 EDA | 30 | 0.336 | 0.022 | 6.4 | 69.7 | 0.740 vs cells with sucrose |
| Cells with 0.3 M sucrose pretreated with 0.42 EDA | 29 | 0.380 | 0.024 | 6.3 | 78.9 | **6.0E-15** vs cells with sucrose |
| Cells with 0.3 M sucrose pretreated with 1.05 EDA | 30 | 0.375 | 0.017 | 4.4 | 77.8 | **3.1E-15** vs cells with sucrose |
| Cells with 0.3 M sucrose pretreated with 2.1 EDA | 29 | 0.384 | 0.023 | 6.0 | 79.7 | **8.6E-17** vs cells with sucrose |

| | **n** | **x̅** | **SD** | **CV** | **% surv.** | **P** |
|---|---|---|---|---|---|---|
| Sucrose-free cells | 59 | 0.657 | 0.032 | 4.9 | 100.0 | |
| Cells with 0.3 M sucrose | 59 | 0.463 | 0.040 | 8.6 | 70.5 | **7E-55** vs sucrose-free control cells (CC) |
| Cells with 0.3 M sucrose pretreated with 0.21 EDA | 30 | 0.440 | 0.039 | 8.8 | 67.0 | 0.011 vs cells with sucrose |
| Cells with 0.3 M sucrose pretreated with 0.42 EDA | 29 | 0.474 | 0.044 | 9.3 | 72.3 | 0.216 vs cells with sucrose |
| Cells with 0.3 M sucrose pretreated with 1.05 EDA | 30 | 0.488 | 0.035 | 7.1 | 74.3 | **0.004** vs cells with sucrose |
| Cells with 0.3 M sucrose pretreated with 2.1 EDA | 29 | 0.513 | 0.031 | 6.1 | 78.1 | **6E-08** vs cells with sucrose |

As can be seen, pretreatment with EDA is capable of significantly protecting against cell death induced by hyperosmotic shock.

### Conclusions:

The results shown demonstrate that pretreatment with EDA is capable of protecting against dehydration and hyperosmotic shock.

### Example 2 - Protective effect of pretreatment with EDA against environmental thermal stress

The object of this experiment is to evaluate the possible protective effect of pretreatment with *Deschampsia antarctica* extract in immortalized human keratinocyte cell line HaCaT against thermal stress (either cold or hot), evaluating said effect firstly with respect to survival and secondly with respect to the action thereof on mitochondrial potential.

### Materials and methods:

### - Products to be tested:

Lyophilized *Deschampsia antarctica* extract (EDA), batch 13E04. The product was directly dissolved in a standard buffer (see below) at a concentration of 1 mg/ml and 100 µl from this solution were applied.

*Deschampsia antarctica* extract (EDA) dried with starch, batch 200314. EDA content 0.25 g EDA/g product. The product was suspended in a standard buffer at a concentration of 10 mg/ml (equivalent to 2.5 mg of EDA/ml), stirred in a magnetic stirrer for 30 minutes and filtered through a 0.2 µm sterile cellulose acetate filter (Minisart, Sartorius). 100 µl from this solution were applied.

### - Culture media and reagents:

RPMI1640 medium (Sigma). Penicillin-Streptomycin (Sigma). Glutamine (Sigma). Fetal calf serum (Sigma). 0.25% Trypsin-EDTA (Sigma). Phosphate-buffered saline (PBS). Standard buffer for test (NaCl 80 mM, KCI 75 mM, D-glucose 25 mM, (4-(3-hydroxy ethyl)-1-piperazine ethanesulphonic acid (Hepes) 25 mM pH 7.4). Tetramethylrhodamine methyl ester perchlorate (TMRM) (Sigma). Carbonyl cyanide m-chlorophenyl hydrazone (CCCP) (Sigma). Glutaraldehyde (Sigma). Crystal violet (Sigma). Sodium dodecyl sulfate (SDS) (Sigma).

### - Cell culture:

The cells used was the immortalized human keratinocyte cell line HaCaT. The cells were cultured in RPMI supplemented with 2 mM glutamine, 50 µg/ml penicillin, 50 U/ml streptomycin, 10% fetal calf serum in plastic 96-well plates with flat bottom special for fluorescence (Nunc), in a Heraeus oven with an atmosphere of 5% CO₂ and 95% humidified air at 37°C. The cells were grown to 100% confluence before treatment. Rows A and H are usually left without cells.

### - Treatment with product:

Thermal stress experiments: The medium was removed and the plates were washed once with 100 µl of standard buffer. 100 µl of EDA solution were added and the plates were incubated for 1 hour at 37°C in an oven under the conditions indicated for cell culture. Three plates were used per experiment, one for control without thermal attack, another for cold thermal attack and the last one for heat thermal attack; half of each plate was dedicated to control and the other half to pretreatment with EDA.

Thermal attack: The medium was removed (+/- product), the plate was washed twice with 100 µl of standard buffer and 100 µl of standard buffer per well were added to the entire plate. The Control Plate was not incubated (i.e., it was kept in the oven under normal conditions), the "Cold" Plate was stored in a freezer at -20°C or at -35°C for 40 minutes and the "Hot" Plate was incubated in an oven at 45°C for 2 hours or at 42°C for 45 or 60 minutes. The standard buffer was removed, the plate was washed with tempered standard buffer 2 x 100 µl per well, 100 µl of tempered standard buffer per well were applied and the plate was left to acclimatize for 15 minutes at 37°C, and the mitochondrial potential and then the cell viability were measured.

### - Measuring mitochondrial potential:

This was performed according to the protocol described by Huang (Huang SG. Development of a High Throughput Screening Assay for Mitochondrial Membrane Potential in Living Cells. J Biomol Screen 2002, 7: 383-9): The plate was washed twice with standard buffer and 100 µl of standard buffer per well were added to the entire plate and 10 µl of TMRM per well (from a 1.65 µM solution in standard buffer, prepared extemporaneously from a 0.8 mM solution in DMSO) were added to the entire plate and 10 µl of CCCP per well (from a 0.12 mM solution in standard buffer, prepared extemporaneously from a 10 mM solution in DMSO) were added to the corresponding wells (usually half of the plate). The plate was incubated for 15 minutes under the conditions indicated above. Once the incubation has ended, the plate was washed 4 times with 160 µl of PBS per well. 100 µl of PBS per well were added and the plate was read in a bottom-reading BMG Fluostar Optima fluorescence reader, using a 530 nm excitation filter and a 590 nm emission filter.

### - Measuring cell viability:

The plates were washed twice with PBS. They were fixed with 100 µl of a 0.25% glutaraldehyde solution for 15 minutes at room temperature. They were stained with a 0.2% crystal violet solution in a 2% ethanol solution for 10 minutes at 37°C. The plate was washed with water and the cells were solubilized with 1% sodium dodecyl sulfate (SDS). The plate was carefully stirred in a plate stirrer and the absorbance at 540 nm was read in a BMG Fluostar Optima spectrophotometer.

### - Statistical analysis:

Statistical analysis was performed for each experiment, considering the value of each well as an independent value, determining the significance by means of Student's t-test using Excel or Graph Pad Prism.

### Results

### - Cold thermal stress:

### a) Cell survival:

The results from two experiments are shown in the following tables:

| **Viability (crystal violet)** | **x̅** | **SD** | **n** | **P** | | **% vs CC** | **EDA / CC thereof** |
|---|---|---|---|---|---|---|---|
| Control cells (CC) | 1.012 | 0.078 | 36 | | | 100 | |
| EDA 1 mg/ml | 1.243 | 0.081 | 36 | **<0.0001** | CC vs EDA | 123 | 1.23 |
| CC + cold (-20°C, 40 min) (F) | 1.036 | 0.107 | 36 | 0.282 | CC vs CC+F | 102 | |
| EDA 1 mg/ml + F | 1.177 | 0.038 | 33 | **<0.0001** | CC+F vs EDA+F | 116 | 1.14 |

| **Viability (crystal violet)** | **x̅** | **SD** | **n** | **p** | | **% vs CC** | **EDA / CC thereof** |
|---|---|---|---|---|---|---|---|
| Control cells (CC) | 0.734 | 0.102 | 36 | | | 100 | |
| EDA 1 mg/ml | 1.054 | 0.098 | 36 | **<0.001** | CC vs EDA | 144 | 1.44 |
| CC + cold (-20°C, 40 min) (F) | 0.799 | 0.086 | 36 | ns | CC vs CC+F | 109 | |
| EDA 1 mg/ml + F | 1.038 | 0.168 | 33 | **<0.001** | CC+F vs EDA+F | 141 | 1.30 |

As can be seen, cold thermal stress does not cause a reduction in cell viability under the indicated conditions and a clear proliferative effect of EDA is indeed observed.

Under more severe conditions (in which freezing has occurred), the effect on cell viability is much more significant and the protective effect of EDA is clearly observed:

| **Viability (crystal violet)** | **x̅** | SD | n | **p** | | **% vs** CC | EDA / CC |
|---|---|---|---|---|---|---|---|
| Control cells CC | 2.142 | 0.294 | 34 | | | 100.0 | |
| EDA 1 mg/ml | 1.937 | 0.537 | 30 | 0.060 | CC vs EDA | 90.5 | 0.90 |
| CC + cold (-20°C, 40 min) (F) | 0.489 | 0.700 | 36 | **<0.0001** | CC+F vs CC | 22.8 | |
| EDA 1 mg/ml + F | 0.826 | 0.521 | 36 | **0.023** | CC+F vs EDA+F | 38.6 | 1.69 |

### b) Mitochondrial potential and corrected mitochondrial potential:

In view of the preceding result, a decision was made to calculate not only the mitochondrial potential, but also the corrected mitochondrial potential for viable cells, considering that the latter may be more representative of the biological phenomenon. The following tables show the results obtained in the experiments at -20°C:

| **Mitochondrial potential** (Δψm) | **x̅** | **SD** | **n** | **p** | | **% vs CC** | **EDA/CC thereof** |
|---|---|---|---|---|---|---|---|
| Control cells (CC) | 15053 | 2024 | 18 | | | 100 | |
| EDA 1 mg/ml | 19080 | 4503 | 18 | **0.001** | CC vs EDA | 127 | 1.27 |
| CC + cold (-20°C, 40 min) (F) | 5153 | 2964 | 17 | **<0.0001** | CC+F vs CC | 34 | |
| EDA 1 mg/ml + F | 11023 | 2140 | 16 | **<0.0001** | CC+F vs EDA+F | 73 | 2.14 |
| | | | | | | | |

| **Corrected Δψm (=Δψm/viable cells)** | **x̅** | **SD** | **n** | **p** | | **% vs CC** | **EDA vs CC thereof** |
|---|---|---|---|---|---|---|---|
| CC | 14869 | 3150 | 18 | | | 100 | |
| EDA 1 mg/ml | 15355 | 4624 | 18 | 0.715 | CC vs EDA | 103 | 1.03 |
| CC + cold (-20°C, 40 min) (F) | 4972 | 3373 | 17 | **<0.0001** | CC vs CC+F | 33 | |
| EDA 1 mg/ml + F | 9364 | 2121 | 16 | **0.0001** | CC+F vs EDA+F | 63 | 1.88 |

| **Mitochondrial potential** (Δψm) | **x̅** | **SD** | **n** | **p** | | **% vs CC** | **EDA/CC thereof** |
|---|---|---|---|---|---|---|---|
| CC | 12991 | 6596 | 17 | | | 100 | |
| EDA 1 mg/ml | 11401 | 3439 | 18 | 0.407 | CC vs EDA | 88 | 0.88 |
| CC + cold (-20°C, 40 min) (F) | 4503 | 4116 | 18 | **<0.001** | CC+F vs CC | 35 | |
| EDA 1 mg/ml + F | 8003 | 2728 | 18 | **0.005** | CC+F vs EDA+F | 62 | 1.78 |
| | | | | | | | |

| **Corrected Δψm (=Δψm/viable cells)** | **x̅** | **SD** | **n** | **p** | | **% vs CC** | **EDA / CC thereof** |
|---|---|---|---|---|---|---|---|
| CC | 17704 | 11447 | 17 | | | 100 | |
| EDA 1 mg/ml | 10818 | 4266 | 18 | **0.023** | CC vs EDA | 61 | 0.61 |
| CC + cold (-20°C, 40 min) (F) | 5639 | 5761 | 18 | **0.0004** | CC vs CC+F | 32 | |
| EDA 1 mg/ml + F | 7713 | 3881 | 18 | 0.214 | CC+F vs EDA+F | 44 | 1.37 |

As can be seen, the cold causes a clear reduction in mitochondrial membrane potential, which reduction reverted by EDA; in the case of corrected mitochondrial potential, the cold also causes a significant reduction and EDA shows a clear protective tendency.

It is not possible to measure mitochondrial potential in more aggressive conditions.

### - Heat thermal stress:

### a) Cell survival:

The results are shown in the following tables:

| **Viability (crystal violet)** | **x̅** | **SD** | **n** | **p** | | **% vs CC** | **EDA vs CC thereof** |
|---|---|---|---|---|---|---|---|
| Control cells (CC) | 1.012 | 0.078 | 36 | | | 100 | |
| EDA 1 mg/ml | 1.243 | 0.081 | 36 | **<0.0001** | CC vs EDA | 123 | 1.23 |
| CC + heat (45°C, 2 hours) (Q) | 0.108 | 0.033 | 36 | **<0.0001** | CC vs CC+Q | 11 | |
| EDA 1 mg/ml + Q | 0.453 | 0.116 | 35 | **<0.0001** | CC+Q vs EDA+Q | 45 | 4.20 |

| **Viability (crystal violet)** | **x̅** | **SD** | **n** | **p** | | **% vs CC** | **EDA / CC thereof** |
|---|---|---|---|---|---|---|---|
| Control cells (CC) | 0.734 | 0.102 | 36 | | | 100 | |
| EDA 1 mg/ml | 1.054 | 0.098 | 36 | **<0.0001** | CC vs EDA | 144 | 1.44 |
| CC + heat (45°C, 2 hours) (Q) | 0.332 | 0.071 | 35 | **<0.0001** | CC vs CC+Q | 45 | |
| EDA 1 mg/ml + Q | 0.735 | 0.142 | 36 | **<0.0001** | CC+Q vs EDA+Q | 100 | 2.21 |

As can be seen, the heat (45°C, 2 hours) causes a significant decline in cell viability, which decline is reverted by pretreatment with EDA.

A smaller decline in cell viability is observed in milder conditions (42°C, 45 minutes), which decline is, as in the preceding case, reverted by pretreatment with EDA.

| **Viability (crystal violet)** | **x̅** | **SD** | **n** | **p** | | **% vs CC** | **EDA / CC thereof** |
|---|---|---|---|---|---|---|---|
| Control cells (CC) | 1.727 | 0.091 | 33 | | | 100.0 | |
| EDA 1 mg/ml | 1.963 | 0.081 | 34 | **<0.0001** | CC vs EDA | 113.7 | 1.14 |
| CC + heat (42°C, 45 min) (Q) | 1.556 | 0.350 | 35 | **0.008** | CC+Q vs CC | 90.1 | |
| EDA 1 mg/ml + Q | 1.738 | 0.155 | 35 | **0.006** | CC+Q vs EDA+Q | 100.7 | 1.12 |

| **Viability (crystal violet)** | **x̅** | **SD** | **n** | **p** | | **% vs CC** | **EDA / CC thereof** |
|---|---|---|---|---|---|---|---|
| Control cells (CC) | 1.352 | 0.119 | 48 | | | 100 | |
| EDA 2.5 mg/ml | 1.698 | 0.166 | 48 | **<0.0001** | CC vs EDA | 126 | 1.26 |
| CC + heat (42°C, 45 min) (Q) | 0.932 | 0.098 | 48 | **<0.0001** | CC vs CC+Q | 69 | |
| EDA 2.5 mg/ml + Q | 1.151 | 0.084 | 48 | **<0.0001** | CC+Q vs EDA+Q | 85 | 1.24 |

The effect of EDA is the same in slightly harsher conditions (42°C, 60 min):

| **Viability (crystal violet)** | **x̅** | **SD** | **n** | **p** | | **% vs CC** | **EDA / CC thereof** |
|---|---|---|---|---|---|---|---|
| CC | 0.694 | 0.133 | 48 | | | 100 | |
| EDA 2.5 mg/ml | 1.356 | 0.260 | 48 | **<0.0001** | CC vs EDA | 195 | 1.95 |
| CC + heat (42°C, 60 min) (Q) | 0.336 | 0.051 | 32 | **<0.0001** | CC vs Heat | 48 | |
| EDA 2.5 mg/ml + Q | 1.205 | 0.362 | 45 | **<0.0001** | Heat 60 min vs Heat 60 min + EDA | 174 | 3.59 |

### b) Mitochondrial potential and corrected mitochondrial potential:

In the first tested conditions (45°C, 2 hours) it is not possible to reliably detect mitochondrial potential in control groups or in treated groups.

In milder conditions (42°C, 45 minutes), it is observed that EDA has a clear tendency to protect mitochondrial potential from thermal stress caused by heat:

| **Mitochondrial potential** (Δψm) | **x̅** | **SD** | **n** | **p** | | **% vs CC** | **EDA/CC thereof** |
|---|---|---|---|---|---|---|---|
| Control cells (CC) | 12787 | 2617 | 16 | | | 100.0 | |
| EDA 1 mg/ml | 14388 | 5503 | 18 | 0.297 | CC vs EDA | 112.5 | 1.13 |
| CC + heat (42°C, 45 min) (Q) | 7010 | 4141 | 15 | **<0.0001** | CC+Q vs CC | 54.8 | |
| EDA 1 mg/ml + Q | 11188 | 4816 | 16 | **0.015** | CC+Q vs EDA+Q | 87.5 | 1.60 |
| | | | | | | | |

| **Corrected Δψm (=Δψm/viable cells)** | **x̅** | **SD** | **n** | **p** | | **% vs CC** | **EDA/CC thereof** |
|---|---|---|---|---|---|---|---|
| Control cells (CC) | 7406 | 1907 | 16 | | | 100.0 | |
| EDA 1 mg/ml | 7331 | 3108 | 18 | ns | CC vs EDA | 99.0 | 0.99 |
| CC + heat (42°C, 45 min) (Q) | 4506 | 3677 | 15 | **0.009** | CC vs CC+Q | 60.8 | |
| EDA 1 mg/ml + Q | 6437 | 3344 | 16 | 0.137 | CC+Q vs EDA+Q | 86.9 | 1.43 |

| **Mitochondrial potential** (Δψm) | **x̅** | **SD** | **n** | **p** | | **% vs CC** | **EDA/CC thereof** |
|---|---|---|---|---|---|---|---|
| Control cells (CC) | 11983 | 5099 | 24 | | | 100 | |
| EDA 2.5 mg/ml | 11363 | 3870 | 24 | 0.660 | CC vs EDA | 95 | 0.95 |
| CC + heat (42°C, 45 min) (Q) | 7299 | 2841 | 24 | **0.0003** | CC vs CC+Q | 61 | |
| EDA 2.5 mg/ml + Q | 8497 | 1695 | 24 | 0.083 | CC+Q vs EDA+Q | 71 | 1.16 |
| | | | | | | | |

| **Corrected Δψm (=Δψm/viable cells)** | **x̅** | **SD** | **n** | **p** | | **% vs CC** | **EDA/CC thereof** |
|---|---|---|---|---|---|---|---|
| Control cells (CC) | 8862 | 4553 | 24 | | | 100 | |
| EDA 2.5 mg/ml | 6692 | 2934 | 24 | 0.056 | CC vs EDA | 76 | 0.76 |
| CC + heat (42°C, 45 min) (Q) | 7834 | 3875 | 24 | 0.404 | Q vs EDA+Q | 88 | |
| EDA 2.5 mg/ml + Q | 7381 | 2008 | 24 | 0.614 | EDA vs EDA+Q | 83 | 0.94 |

The effect is the same although clearer in slightly harsher conditions (42°C, 60 min):

| **Mitochondrial potential** (Δψm) | **x̅** | **SD** | **n** | **p** | | **% vs CC** | **EDA/CC thereof** |
|---|---|---|---|---|---|---|---|
| Control cells (CC) | 10630 | 4967 | 24 | | | 100 | |
| EDA 2.5 mg/ml | 8639 | 2549 | 24 | 0.086 | CC vs EDA | 81 | 0.81 |
| CC + heat (42°C, 60 min) (Q) | 4672 | 3526 | 12 | **0.001** | CC vs Q | 44 | |
| EDA 2.5 mg/ml + Q | 9711 | 3531 | 22 | **0.0003** | Q vs EDA + Q | 91 | 2.08 |
| | | | | | | | |

| **Corrected Δψm (=Δψm/viable cells)** | **x̅** | **SD** | **n** | **p** | | **% vs CC** | **EDA / CC thereof** |
|---|---|---|---|---|---|---|---|
| Control cells (CC) | 15314 | 10086 | 24 | | | 100 | |
| EDA 2.5 mg/ml | 6372 | 3103 | 24 | **0.0001** | CC vs EDA | 42 | 0.42 |
| CC + heat (42°C, 60 min) (Q) | 13916 | 12621 | 12 | 0.7208 | CC+Q vs CC | 91 | |
| EDA 2.5 + Q mg/ml | 8061 | 5354 | 22 | 0.0662 | Q vs EDA + Q | 53 | 0.58 |

### Conclusions:

The results shown demonstrate that pretreatment with EDA is capable of protecting against the effects of cold and heat thermal stress.

### Example 3 - Protective effect of pretreatment with EDA against environmental pollution (tobacco smoke)

The object of this experiment is to evaluate the possible protective effect of pretreatment with *Deschampsia antarctica* extract in human fibroblasts or transformed keratinocytes against environmental contamination such as that caused by tobacco smoke, evaluating said effect on cell survival.

### Materials and methods:

### - Products to be tested:

*Deschampsia antarctica* extract (EDA): In experiments with fibroblasts, EDA dried with starch, batch 150414, was used. EDA content 0.25 g EDA/g product. The product was suspended in a complete DMEM medium at a concentration of 10 mg/ml (equivalent to 2.5 mg of EDA/ml), stirred in a magnetic stirrer for 30 minutes and filtered through a 0.2 µm sterile cellulose acetate filter (Minisart, Sartorius). In experiments with keratinocytes, EDA adsorbed on maltodextrin batch 160215 (dry green leaf batch 241114) was used. The product was dissolved in a DMEM medium at a concentration of 10 mg/ml (equivalent to 2.1 mg of EDA/ml), stirred in a magnetic stirrer for 30 minutes and filtered through a 0.2 µm sterile cellulose acetate filter (Minisart, Sartorius).

Tobacco extract (CSC) extemporaneously prepared according to Lamb *et al* 2012 (Lamb DJ, Parker N, Ulrich K, Walsh R, Yeadon M, Evans SM. Characterization of a Mouse Model of Cigarette Smoke Extract-Induced Lung Inflammation. J Pulmon Resp Med 2012, 2:125) with slight modifications: a cigarette with filter (Ducados brand) was connected to the end of a peristaltic pump, the pump was put into operation, the cigarette was lighted up and the smoke was drawn in by means of the peristaltic pump, making it bubble in a test tube containing 20 ml of phenol red-free DMEM medium (5% CSC) or 7.5 cigarettes in 15 ml (50% CSC). Each cigarette lasted for an average time of 7 minutes.

### - Culture media and reagents:

Phenol red-free DMEM medium (Sigma). DMEM medium (Sigma). Penicillin-Streptomycin (Sigma). Glutamine (Sigma). Fetal calf serum (Sigma). 0.25% Trypsin-EDTA (Sigma). Phosphate-buffered saline (PBS). Hank's balanced salt solution (hereinafter Hank's) (Sigma). Glutaraldehyde (Sigma). Crystal violet (Sigma). Sodium dodecyl sulfate (SDS) (Sigma).

### - Fibroblast cell culture:

The cells used were human dermal fibroblasts isolated from the outer area of the retroauricular flap obtained from different healthy voluntary donors and preserved in liquid N₂. The experiments used cells grown in culture flasks containing DMEM supplemented with 2 mM glutamine, 50 µg/ml penicillin, 50 U/ml streptomycin, 10% fetal bovine serum, in a Heraeus oven with an atmosphere of 7% CO₂ and 93% humidified air at 37°C, until reaching about 90% of confluence, after which a new pass was performed by means of trypsinization. For the experiments, cells between passes 3 and 6 which were seeded in 6- or 96-well plates with flat bottom (Nunc) at an initial density of 10⁵ cells/ml, respectively, were used and were left to grow in an incubator until reaching confluence.

### - Keratinocyte cell culture:

The cells used was the immortalized human keratinocyte cell line HaCaT. The cells were cultured in DMEM supplemented with 2 mM glutamine, 50 µg/ml penicillin, 50 U/ml streptomycin, 10% fetal calf serum in plastic 96-well plates with flat bottom (Nunc), in a Heraeus oven with an atmosphere of 5% CO₂ and 95% humidified air at 37°C. For the experiments, cells which were seeded in 96-well plates with flat bottom (Nunc) at an initial density of 105 cells/ml, respectively, were used and were left to grow in an incubator until reaching confluence.

### - Treatment with products:

Pretreatment with EDA: Both in the experiments with fibroblasts and in those with keratinocytes, 100 µl per well of the EDA solution prepared as indicated above were added to half of each plate and the plate was incubated for about 20 hours in an incubator.

Treatment with CSC: In the experiments with fibroblasts, after pretreatment, the plates were washed twice with Hank's and 100 µl per well of the 5% CSC solution prepared as indicated above or 100 µl per well of phenol red-free DMEM solution were added and the plates were incubated for 3.5 hours in an incubator.

In the experiments with keratinocytes, after pretreatment, the plates were washed twice with Hank's and 100 µl per well of the 50% CSC solution prepared as indicated above or 100 µl per well of phenol red-free DMEM solution were added and the plates were incubated for 20 hours in an incubator.

The amounts indicated are for 96-well plates. For 6-well plates (only in the case of fibroblasts), the same method is used, multiplying the amount x30.

### - Measuring cell viability:

The 96-well plates were washed twice with Hank's. They were fixed with 100 µl of a 0.25% glutaraldehyde solution in Hank's for 15 minutes at room temperature. They were washed once with water. They were stained with a 0.2% crystal violet solution in a 2% ethanol solution for 15 minutes at 37°C. The plate was washed with water and the cells were solubilized with 100 µl of 1% sodium dodecyl sulfate (SDS). It was carefully stirred in a plate stirrer and the absorbance at 550 nm was read in a BMG Fluostar Optima spectrophotometer.

The similar method is used for 6-well plates, multiplying the volumes by 30. In this case, the fixed and stained cells were not solubilized and photographs of said cells were taken. Microphotographs were also taken with an Olympus OM-2 camera coupled to an Olympus IMT-311 inverted microscope by means of an FK eyepiece adaptor and an adaptor for microphotography L. A 400 ASA Kodak color plus film roll was used.

### - Statistical analysis:

Statistical analysis was performed for each experiment, considering the value of each well as an independent value, determining the significance by means of Student's t-test using Excel or Graph Pad Prism.

### Results:

### a) Experiments with fibroblasts:

The following table shows the result of three different experiments.

| | **x̅** | **SD** | **n** | **p** | | **% viability vs CC** |
|---|---|---|---|---|---|---|
| Control cells (CC) | 0.686 | 0.037 | 48 | | | 100.0 |
| EDA | 0.649 | 0.029 | 48 | **<0.0001** | CC vs EDA | 94.5 |
| | | | | | | |
| CC + 5% CSC | 0.360 | 0.040 | 48 | **<0.0001** | CC vs CC+CSC | 52.5 |
| EDA + 5% CSC | 0.521 | 0.034 | 48 | **<0.0001** | CC+CSC vs EDA+CSC | 75.9 |
| | | | | **<0.0001** | EDA vs EDA+CSC | |

| | **x̅** | **SD** | **n** | **p** | | **% viability vs CC** |
|---|---|---|---|---|---|---|
| CC | 0.524 | 0.025 | 48 | | | 100.0 |
| EDA | 0.538 | 0.018 | 48 | **0.002** | CC vs EDA | 102.7 |
| | | | | | | |
| CC + 5% CSC | 0.466 | 0.029 | 48 | **<0.0001** | CC vs CC+CSC | 89.0 |
| EDA + 5% CSC | 0.552 | 0.026 | 48 | **<0.0001** | CC+CSC vs EDA+CSC | 105.3 |
| | | | | 0.696 | EDA vs EDA+CSC | |

| | **x̅** | **SD** | **n** | **p** | | **% viability vs CC** |
|---|---|---|---|---|---|---|
| CC | 0.417 | 0.046 | 48 | | | 100.0 |
| EDA | 0.386 | 0.019 | 48 | **<0.0001** | CC vs EDA | 92.6 |
| | | | | | | |
| CC + 5% CSC | 0.262 | 0.016 | 48 | **<0.0001** | CC vs CC+CSC | 62.7 |
| EDA + 5% CSC | 0.325 | 0.018 | 48 | **<0.0001** | CC+CSC vs EDA+CSC | 77.8 |
| | | | | **<0.0001** | EDA vs EDA+CSC | |

As can be seen, treatment with tobacco smoke (5% CSC) causes a reduction in average fibroblast viability of 32% and this reduction goes down to 18% when pretreated with EDA; i.e., there is a reduction of 44% in the mortality induced by tobacco smoke.

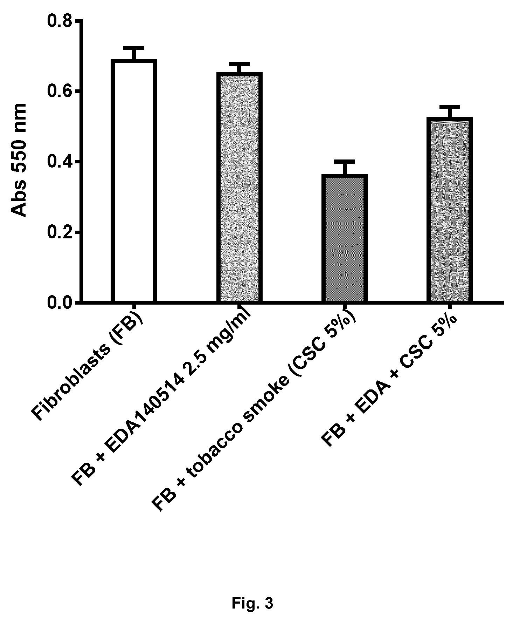

Figure 3 shows as an example the results of one of the experiments (the differences among the different groups are significant (p<0.01)).

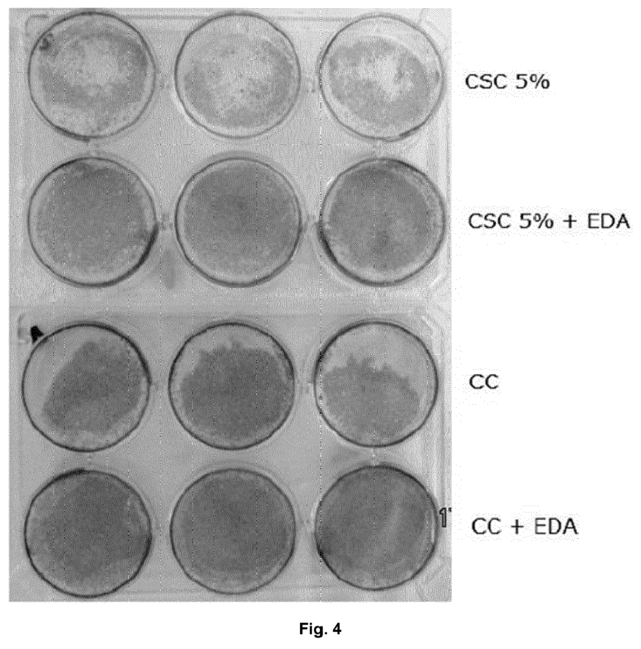

The same effect can be observed in 6-well plates (Figure 4).

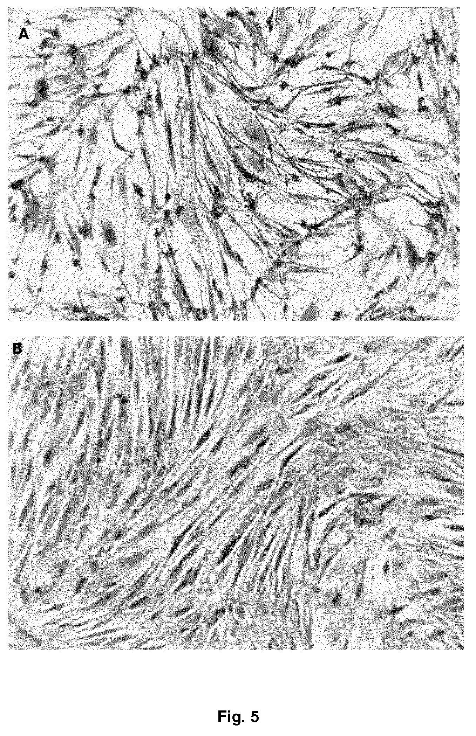

Figure 5 shows the differences in cell morphology where, besides a reduction in the number of cells (lower density), treatment with tobacco smoke (A) is shown to cause a highly disordered arrangement of cells as well as cells with non-fusiform, abnormal shapes, some with jagged edges, whereas cells pretreated with EDA and then with tobacco smoke (B) show the characteristic fusiform shape of fibroblasts and a great organization which is also characteristic of fibroblasts.

### b) Experiments with keratinocytes:

The following table shows the result of two different experiments.

| X | **x̅** | **SD** | **n** | **p** | | **% viability vs CC** |
|---|---|---|---|---|---|---|
| Control cells (CC) | 1.380 | 0.143 | 30 | | | 100 |
| EDA | 1.481 | 0.138 | 30 | **0.007** | EDA vs CC | 107 |
| 50% CSC | 1.015 | 0.085 | 30 | **<0.0001** | CSC vs CC | 74 |
| 50% CSC + EDA | 1.313 | 0.076 | 30 | **<0.0001** | EDA + CSC vs CSC | 95 |
| | | | | **<0.0001** | EDA + CSC vs EDA | |
| | | | | **<0.0001** | CSC + EDA vs CC | |

| | **x̅** | **SD** | **n** | **p** | | **% viability vs CC** |
|---|---|---|---|---|---|---|
| Control cells (CC) | 1.271 | 0.103 | 30 | | | 100 |
| EDA | 1.283 | 0.128 | 30 | 0.688 | EDA vs CC | 101 |
| 50% CSC | 0.876 | 0.074 | 30 | **<0.0001** | CSC vs CC | 69 |
| 50% CSC + EDA | 1.166 | 0.157 | 30 | **<0.0001** | EDA + CSC vs CSC | 92 |
| | | | | **0.003** | EDA + CSC vs EDA | |
| | | | | **0.003** | CSC + EDA vs CC | |

As can be seen, treatment with tobacco smoke (50% CSC) causes a reduction in average keratinocyte viability of 28% and this reduction goes down to 6% when pretreated with EDA; i.e., there is a reduction of 79% in the mortality induced by tobacco smoke.

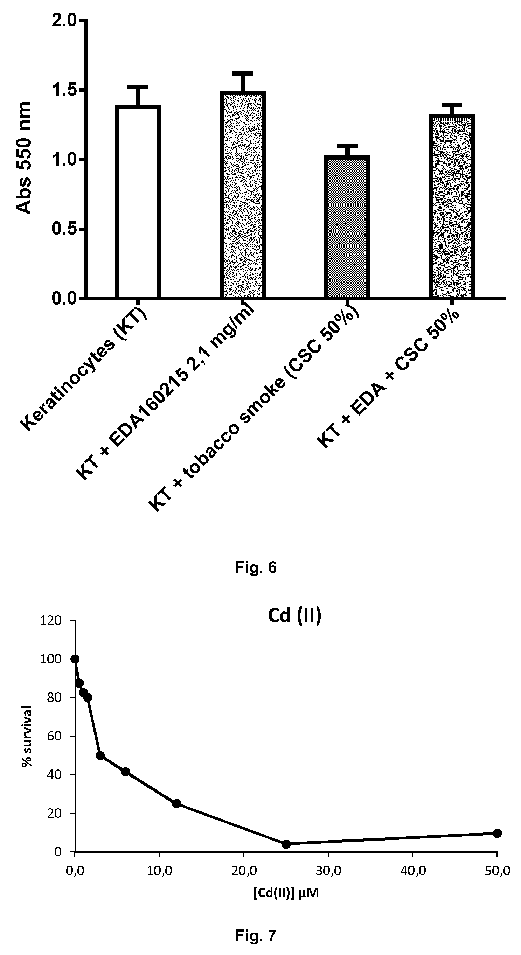

Figure 6 shows as an example the results of one of the experiments (the differences among the different groups are significant (p<0.01)).

### Conclusions:

The results shown demonstrate that pretreatment with EDA is capable of protecting against the effects of tobacco smoke pollution.

### Example 4 - Protective effect of pretreatment with EDA against environmental pollution (arsenic, cadmium, chromium)

The object of this experiment is to evaluate the possible protective effect of pretreatment with *Deschampsia antarctica* extract in human fibroblasts against environmental contamination such as that caused by arsenic, cadmium or chromium, evaluating said effect on cell survival.

### Materials and methods:

### - Products to be tested:

*Deschampsia antarctica* extract (EDA): In experiments with fibroblasts, lyophilized EDA batch 051214 was used. The product was dissolved in a complete DMEM medium at a concentration of 5 mg/ml, stirred in a magnetic stirrer for 30 minutes and filtered through a 0.2 µm sterile cellulose acetate filter (Minisart, Sartorius).

Cd(II): cadmium chloride (CdCl₂) at a final concentration of 0.3 mM was dissolved in phenol red-free DMEM medium and filtered through a 0.2 µm sterile cellulose acetate filter (Minisart, Sartorius). The dilutions indicated in the Results section were prepared from this solution with phenol red-free DMEM medium.

Cr(VI) and Cr(III): chromium trioxide (CrO₃) and chromium chloride (CrCl₃) at a final concentration of 0.5 mM were dissolved separately in phenol red-free DMEM medium; equal volumes of both solutions were combined and filtered through a 0.2 µm sterile cellulose acetate filter (Minisart, Sartorius). The dilutions indicated in the Results section were prepared from this solution with phenol red-free DMEM medium.

As(III): sodium arsenite (NaAsO₂) at a final concentration of 9 mM was dissolved in phenol red-free DMEM medium and filtered through a 0.2 µm sterile cellulose acetate filter (Minisart, Sartorius).

### - Culture media and reagents:

Phenol red-free DMEM medium (Sigma). DMEM medium (Sigma). Penicillin-Streptomycin (Sigma). Glutamine (Sigma). Fetal calf serum (Sigma). 0.25% Trypsin-EDTA (Sigma). Phosphate-buffered saline (PBS). Hank's balanced salt solution (hereinafter Hank's) (Sigma). Glutaraldehyde (Sigma). Crystal violet (Sigma). Sodium dodecyl sulfate (SDS) (Sigma).

### - Fibroblast cell culture:

The cells used were human dermal fibroblasts isolated from the outer area of the retroauricular flap obtained from different healthy voluntary donors and preserved in liquid N₂. The experiments used cells grown in culture flasks containing DMEM supplemented with 2 mM glutamine, 50 µg/ml penicillin, 50 U/ml streptomycin, 10% fetal bovine serum, in a Heraeus oven with an atmosphere of 7% CO₂ and 93% humidified air at 37°C, until reaching about 90% of confluence, after which a new pass was performed by means of trypsinization. For the experiments, cells between passes 9 and 11 which were seeded in 96-well plates with flat bottom (Nunc) at an initial density of 10⁵ cells/ml, respectively, were used and were left to grow in an incubator until reaching confluence.

### - Treatment with products:

Pretreatment with EDA: 100 µl per well of the EDA solution prepared as indicated above (or the relevant dilutions in complete DMEM) were added to half of each plate and the plate was incubated for about 24 hours in an incubator.

Treatment with metals: After pretreatment, the plates were washed twice with Hank's and 100 µl per well of the metal-containing solution at the concentration indicated in the Results section prepared as indicated above or 100 µl per well of phenol red-free DMEM solution were added and the plates were incubated for 24 hours in an incubator.

### - Measuring cell viability:

The plates were washed twice with Hank's. They were fixed with 100 µl of a 0.25% glutaraldehyde solution in Hank's for 15 minutes at room temperature. They were washed once with water. They were stained with a 0.2% crystal violet solution in a 2% ethanol solution for 15 minutes at 37°C. The plate was washed with water and the cells were solubilized with 100 µl of 1% sodium dodecyl sulfate (SDS). It was carefully stirred in a plate stirrer and the absorbance at 550 nm was read in a BMG Fluostar Optima spectrophotometer.

### - Statistical analysis:

Statistical analysis was performed for each experiment, considering the value of each well as an independent value, determining the significance by means of Student's t-test using Excel or Graph Pad Prism.

### Results:

### - Effect of pretreatment with EDA with respect to exposure to Cd(II):

The working concentration of CD(II) causing mortality of about 50% was first determined. The results are shown in Figure 7:

As can be seen, Cd(II) at a concentration of 3 µM causes mortality of about 50%.

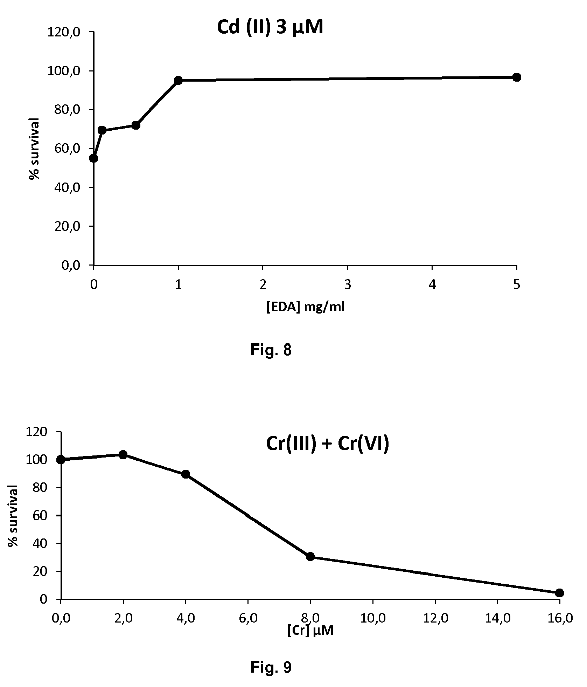

The following table and Figure 8 show the effect of pretreatment with different concentrations of EDA and the subsequent exposition to 3 µM Cd(II).

| **Survival (u abs**) | **n** | **x̅** | **SD** | **CV** | **%** | **p** | |
|---|---|---|---|---|---|---|---|
| CC | 30 | 0.342 | 0.051 | 14.8 | 100.0 | | |
| EDA 5 mg/ml + 3 µM Cd | 12 | 0.330 | 0.044 | 13.4 | 96.6 | **<0.0001** | EDA 5 mg/ml vs 3 µM Cd |
| EDA 1 mg/ml + 3 µM Cd | 18 | 0.325 | 0.048 | 14.7 | 95.0 | **<0.0001** | EDA 1 mg/ml vs 3 µM Cd |
| EDA 0.5 mg/ml + 3 µM Cd | 18 | 0.246 | 0.038 | 15.3 | 71.9 | **<0.0001** | EDA 0.5 mg/ml vs 3 µM Cd |
| EDA 0.1 mg/ml + 3 µM Cd | 11 | 0.237 | 0.046 | 19.4 | 69.3 | **0.008** | EDA 0.1 mg/ml vs 3 µM Cd |
| 3 µM Cd | 29 | 0.188 | 0.050 | 26.3 | 54.9 | **<0.0001** | CC vs 3 µM Cd |

As can be seen, pretreatment with EDA significantly protects all the tested concentrations against subsequent exposure to cadmium chloride.

### - Effect of pretreatment with EDA against exposure to Cr(III) and Cr(VI):

The working concentration of Cr(III) and Cr(VI) was first determined by studying fibroblast survival with respect to different concentrations of these metals. The results are shown in Figure 9:

As can be seen, survival of about 50% is achieved at a concentration of 6 µM, so this concentration was selected for the protection experiments.

The following table shows the results of an experiment consisting of pretreatment with EDA (5 mg/ml) and subsequent exposure to Cr(III)+Cr(VI):

| | **n** | **x̅** | **SD** | **CV** | **%** | **%** | **p** | |
|---|---|---|---|---|---|---|---|---|
| CC | 24 | 0.195 | 0.057 | 29.4 | 100.0 | | | |
| CC + 6 µM Cr | 17 | 0.103 | 0.034 | 33.0 | 52.8 | 100.0 | **<0.0001** | CC vs CC + 6 µM Cr |
| EDA + 6 µM Cr | 18 | 0.131 | 0.025 | 19.2 | 67.1 | 127.2 | **0.0099** | CC + 6 µM Cr vs EDA + 6 µM Cr |

As can be seen, pretreatment with EDA has a significant protective effect against the attack caused by joint exposure to chromium trioxide and chromium chloride.

### - Effect of pretreatment with EDA with respect to exposure to As(III):

The following table shows the result of three experiments studying the effect of pretreatment with EDA (5 mg/ml) against the attack caused by subsequent exposure to 9 mM sodium arsenite (9 mM NaAsO₂).

| **Survival (u abs)** | **n** | **x̅** | **SD** | **CV** | **%** | **%** | **p** | |
|---|---|---|---|---|---|---|---|---|
| CC | 24 | 0.719 | 0.040 | 5.5 | 100.0 | | | |
| 9 mM sodium arsenite | 18 | 0.334 | 0.062 | 18.5 | 46.4 | 100 | **p<0.0001** | CC vs 9 mM AsO₂ |
| EDA + 9 mM sodium arsenite | 18 | 0.455 | 0.047 | 10.2 | 63.2 | 136.3 | **p<0.0001** | 9 mM AsO₂ vs 9 mM AsO₂ + EDA |
| | | | | | | | **p<0.0001** | CC vs 9 mM AsO₂ + EDA |

| | **n** | **x̅** | **SD** | **CV** | **%** | **%** | **p** | |
|---|---|---|---|---|---|---|---|---|
| CC | 24 | 0.498 | 0.045 | 9.1 | 100.0 | | | |
| 9 mM sodium arsenite | 18 | 0.209 | 0.019 | 9.0 | 42.0 | 100 | **p<0.0001** | CC vs 9 mM AsO₂ |
| EDA + 9 mM sodium arsenite | 18 | 0.282 | 0.021 | 7.5 | 56.6 | 134.6 | **p<0.0001** | 9 mM AsO₂ vs 9 mM AsO₂ + EDA |
| | | | | | | | **p<0.0001** | CC vs 9 mM AsO₂ + EDA |

| | **n** | **x̅** | **SD** | **CV** | **%** | **%** | **p** | |
|---|---|---|---|---|---|---|---|---|
| CC | 24 | 0.378 | 0.040 | 10.6 | 100.0 | | | |
| 9 mM sodium arsenite | 17 | 0.206 | 0.024 | 11.6 | 54.6 | 100 | **p<0.0001** | CC vs 9 mM AsO₂ |
| EDA + 9 mM sodium arsenite | 18 | 0.275 | 0.024 | 8.5 | 72.9 | 133.4 | **p<0.0001** | 9 mM AsO₂ vs 9 mM AsO₂ + EDA |
| | | | | | | | **p<0.0001** | CC vs 9 mM AsO₂ |

As can be seen, pretreatment with EDA has a significant protective effect against attack caused by exposure to sodium arsenite.

### Conclusions:

The results shown demonstrate that pretreatment with EDA is capable of protecting against the effects of arsenic, cadmium or chromium pollution.

## Claims

1. Cosmetic use of an Extract of *Deschampsia antarctica* (EDA) for counteracting human skin barrier damage caused by environmental aggressions not derived from UV radiation.

2. The use according claim 1, wherein said extract prevents and/or reduces cutaneous discomfort and cutaneous damage induced by chemical air pollutants and climatic conditions other than UV radiation.

3. The use according to claim 2, wherein the chemical air pollutants are selected from the group consisting of smoke and heavy metals.

4. The use according to claim 2, wherein the climatic conditions are selected from the group consisting of osmotic shocks, low air humidity and environmental thermal stress.

5. Extract of *Deschampsia antarctica* (EDA) for use in the amelioration and/or prevention of human skin barrier disorders and associated signs.

6. EDA for use according to claim 5 wherein the human skin barrier disorder is selected from dry skin, atopic dermatitis and contact dermatitis.

7. EDA for use according to claim 5, wherein the human skin barrier disorder is worsen by environmental aggressions selected from the group consisting of chemical air pollutants and climatic conditions other than UV radiation.

8. Composition for use in the treatment and/or prevention of human skin barrier disorders and associated signs, said composition comprising an effective amount of Extract of *Deschampsia antarctica* (EDA) in a physiologically acceptable medium.
